(19) Europäisches Patentamt
European Patent Office
Office européen des brevets

(11) EP 0 406 695 B1

## (12) EUROPEAN PATENT SPECIFICATION

(45) Date of publication and mention
of the grant of the patent:
**27.05.1998 Bulletin 1998/22**

(51) Int Cl.6: **C11D 3/39**, A61L 2/18, G02C 13/00

(21) Application number: **90112292.9**

(22) Date of filing: **27.06.1990**

(54) **Method for removing soil deposited on a contact lens**

Verfahren zum Entfernen von Schmutz von Kontaktlinsen

Procédé de nettoyage pour enlever les souillures déposées sur une lentille de contact

(84) Designated Contracting States:
**DE FR GB**

(30) Priority: **07.07.1989 JP 176799/89**

(43) Date of publication of application:
**09.01.1991 Bulletin 1991/02**

(73) Proprietor: **Tomei Sangyo Kabushiki Kaisha Nishi-ku, Nagoya-shi, Aichi-ken (JP)**

(72) Inventors:
• **Kato, Takuo, c/o Tomei Sangyo K.K. Nagoya-shi, Aichi-ken (JP)**

• **Nishio, Hitomi, c/o Tomei Sangyo K.K. Nagoya-shi, Aichi-ken (JP)**

(74) Representative:
**Wächtershäuser, Günter, Prof. Dr. Patentanwalt, Tal 29 80331 München (DE)**

(56) References cited:
**EP-A- 0 110 609**      **FR-A- 2 627 084**
**GB-A- 2 173 017**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to a method for removing soil deposited on a contact lens and a cleaning agent (a soil-removing agent) therefore. Particularly, it relates to a technique for efficiently removing soil such as protein soil deposited on or fixed to a lens surface or penetrated into the lens.

Contact lenses presently used, include, for example, hard contact lenses composed essentially of methyl methacrylate, oxygen permeable hard contact lenses composed essentially of siloxanyl methacrylate and soft contact lenses composed essentially of 2-hydroxyethyl methacrylate (HEMA) or N-vinyl-2-pyrrolidone. When put on the eyes, these contact lenses are immersed in vital secretory liquids (lacrimae) whether there are soft or hard. Therefore, when put on the eyes continuously, they will unavoidably be soiled with lipids, proteins, mucins, etc. contained in the vital sectetory liquids. Further, they are likely to be infested with bacteria taking such substances as nutrients. Particularly, water-absorptive soft contact lenses have a problem such that such soil tends to penetrate into the interior of the lens together with water and accumulate therein.

Such soil to the contact lenses tends to fog up the contact lenses and eventually give damages to the eyes. Therefore, cleaning agents for contact lenses are commercially available, and the users are expected to clean the contact lenses before and after the use. In the case of soft contact lenses, heat disinfection is required.

However, commercially available cleaning agents are usually composed mainly of surfactants. Therefore, they are capable of removing lipid soil among soils deposited on contact lenses, but they can not remove proteins or mucins, whereby such protein or mucin soil will remain. Particularly in the case of soft contact lenses, such remaining protein soil will be modified and solidified when the required boiling sterilization is conducted and thus firmly fixed to the lens. Especially, in the case of highly water-absorptive soft contact lenses, protein soil will be fixed also in the interior of the lens, whereby there has been a problem that the lens tends to have a turbidity, and the useful life of the contact lens will thereby be shortened.

As a cleaning agent for the removal of proteins, the one containing a protease and a hydrosulfohydryl compound, has heretofore been known. The soiled lens is immersed in such a cleaning agent, so that the protein soil is thereby decomposed and removed. However, in such a method, it takes a long time for the decomposition of the proteins, and there has been a drawback that the cleaning operation requires a very long time of 6 hours or more.

Further, Japanese Unexamined Patent Publications No. 45399/1984, No. 260214/1986, No. 150318/1987 and No. 47722/1988 disclose protein-removing agents or methods wherein chlorine-type oxidizing agents are employed. However, treating agents using such strong oxidizing agents, have a problem that markings or colored portions of the soft contact lenses tend to be discolored or eliminated. Therefore, they are not useful for colored soft contact lenses or contact lenses marked with dyestuffs.

On the other hand, a number of treating agents and treating methods for contact lenses have been proposed in which various peroxides are used as the oxidizing agents. For example, Japanese PCT Application No. 502245/1987 discloses a solid form disinfecting composition for contact lenses to be added to water, which comprises a disinfectant for contact lenses which is capable of functioning as a hydrogen peroxide supply source, an inactivating agent of a delay release formulation which is capable of making this disinfectant ophthalmologically acceptable and a color indicating reagent which indicates a visible color change at the time of the inactivation. Japanese Examined Patent Publication No. 34343/1986 discloses a disinfecting method of dipping in a bactericidally active aqueous solution containing ascorbic acid, an alkali metal percarbonate and an effective amount of a copper salt catalyst. Further, Japanese Unexamined Patent Publication No. 37313/1988 discloses a bactericidal cleaning system for contact lenses comprising a solid peroxo compound, a reducing agent and/or catalyst, and a surfactant, wherein the peroxo compound-containing component further contains an alkyl glycoxide and a $H_2O_2$ neutralizing component containing a buffering salt to adjust the pH to 7.

However, these treating agents and treating methods are primarily intended for disinfection rather than for removal of protein soil. Even when they have certain effects for removing protein soil as a secondary function, such effects are inadequate for effectively removing the protein soil fixed to contact lenses or the protein soil trapped in the interior of the soft contact lenses.

The present invention has been made under these circumstances. It is an object of the present invention to provide a method for removing soil such as protein soil deposited or fixed on the surface or in the interior of a contact lens efficiently in a short period of time without creating a discoloration problem, and to provide a cleaning agent for such a method.

To solve the above problems, the present invention provides a method for decomposing and removing soil deposited on a contact lens, which comprises immersing the contact lens having soil such as protein soil deposited thereon, in an aqueous solution containing peroxide ions and/or hydrogen peroxide ions and having the pH adjusted to a level of at least 9. Further, in order to more efficiently conduct the cleaning for such soil removal, the aqueous solution in which the contact lens is immersed, may be maintained at a temperature of at least 40°C.

Further, as a cleaning agent useful for such a soil-removing method, (a) a cleaning agent for contact lenses, which consists essentially of an aqueous solution containing peroxide ions and/or hydrogen peroxide ions and having the pH adjusted to a level of at least 9, (b) a cleaning agent for contact lenses, which comprises a peroxide component for generating peroxide ions and/ or hydrogen peroxide ions, and a pH controlling component for adjusting the pH of an aqueous solution having said peroxide component dissolved therein to a desired pH value of at least 9, or (c) a cleaning agent for contact lenses, which is, when dissolved in water, capable of generating peroxide ions and/or hydrogen peroxide ions and giving an aqueous solution having a pH of at least 9, may be selected.

In short, according to the present invention, effective solubilization or dispersion of proteins is conducted by the action of peroxide ions and/or hydrogen peroxide ions present in an aqueous solution in which the contact lens is immersed, whereby removal of protein soil can be completed in a short period of time. The mechanism for the decomposition of proteins has not yet been clearly understood. However, it is considered that the above ions act on the amide to cut the peptide bonds in the protein in the same manner as hydrolysis. Accordingly, in the present invention, the discoloration problem caused by the oxidation can be avoided. Therefore, the present invention can advantageously be applied to colored contact lenses or contact lenses with markings.

Further, such peroxide ions or hydrogen peroxide ions are capable of providing excellent effects at a very low concentration. Accordingly, the aqueous solution, i. e. the cleaning agent, in which the soiled contact lens is immersed, may be prepared at a very low concentration. Therefore, in the cleaning method according to the present invention, preparation of a cleaning solution from the formulated agent and rinsing of the lens after completion of the cleaning, can simply and efficiently be conducted. Thus, the method of the present invention has a merit in that the handling during the cleaning operation is simple.

In addition, the speed for the decomposition of proteins by the peroxide ions and/or hydrogen peroxide ions, can effectively be increased by heating and maintaining the aqueous solution having the contact lens immersed therein at a temperature of at least 40°C during the cleaning, whereby it is possible to further shorten the time for cleaning.

Now, the present invention will be described in detail with reference to the preferred embodiments.

The peroxide ions ($O_2^{2-}$) or hydrogen peroxide ions ($HOO^-$) which are an essential component of the aqueous solution (cleaning solution) to be used in the cleaning method according to the present invention, are generated specifically by e.g. metal peroxides such as sodium peroxide, percarbonates, perborates, persulfates, perphosphates, or hydrogen peroxide compounds such as hydrogen peroxide (aqueous hydrogen peroxide solution) or sodium hydrogen peroxide.

These peroxide components may be used alone or in combination of two or more. The respective cleaning agents of the present invention are prepared using such peroxide components as the main components.

A cleaning solution for contact lenses prepared from such a cleaning agent usually contains peroxide ions and/or hydrogen peroxide ions in an amount within a range of from 0.00001 to 20.0%, preferably within a range of from 0.001 to 10.0%. If the content of such ions is less than 0.00001%, it becomes difficult to obtain adequate effects for removing proteins. On the other hand, if the content exceeds 20.0%, it will be required to carefully conduct the rinsing operation to secure the safety of the lens after the treatment, such being undesirable.

Further, such a cleaning solution is required to be adjusted to a pH value of at least 9.0, preferably about 11, in order to effectively obtain the excellent protein-removal effects. If the pH is less than 9.0, no adequate effects for removing protein soil will be obtained. On the other hand, if the pH is too high, the contact lens itself tends to be deteriorated by alkali. Therefore, the upper limit is preferably at a level of pH13.

In the cleaning method of the present invention, a soiled contact lens is immersed in a cleaning solution having the ion concentration and pH adjusted to meet the above-mentioned conditions, for an effective period of time, followed by a rinsing operation to remove the cleaning solution. To conduct such cleaning more effectively in a short period of time, such a cleaning solution is preferably heated and maintained at a temperature of at least 40°C under a condition in which the soiled contact lens to be cleaned, is immersed. It is common to employ a treating temperature of from 40 to 120°C, more preferably from 50 to 100°C. Further, it is usual to employ a treating time of from 10 to 60 minutes.

The more specific levels of the ion concentration or concentration of the peroxide component as a source for generating such ions, the pH and the treating temperature, may be suitably determined depending upon the type of the peroxide component used, the treating time required and the soiling degree of the lens. However, if all of the concentration of the peroxide component, the pH and the treating temperature are set at high levels, the lens material is likely to deteriorate. On the other hand, if all of them are set at low levels, the decomposition of the protein soil tends to be inadequate. Therefore, it is desired to take a balance inbetween.

For example, in a case where $H_2O_2$ is used as a source for generating hydrogen peroxide ions, a preferred relation among the $H_2O_2$ concentration, the pH and the cleaning treatment temperature is as follows.

Firstly, when the treating temperature is at least 80°C, the pH is preferably from 9.0 to 13.0, and the $H_2O_2$ concentration is preferably within a range of from 0.01 to 1.0%. Particularly preferably, the pH is within a range of from 9.0 to 10.0, and the $H_2O_2$ concentration is from 0.1 to 1.0%. On the other hand, when the pH is within a range of 10.0 to 13.0, the $H_2O_2$ concentration is preferably from 0.01 to 0.1%. Further, in a case where the treating temperature is at least 40°C and less than 80°C, the pH is preferably from 9.0 to 13.0, and the $H_2O_2$ concentration is preferably from 0.01 to 5.0%. More preferably, within the pH range of from 9.0 to 10.0, the $H_2O_2$ concentration is from 1.0 to 5.0%. On the other hand, if the pH is within a range of from 10.0 to 13.0, the $H_2O_2$ concentration is preferably from 0.01 to 1.0%. Further, when the cleaning treatment is conducted at room temperature, it is preferred that the pH is at least 11, and the $H_2O_2$ concentration is at least 1.0%.

The cleaning agent which provides a cleaning solution useful for the cleaning method of the present invention, may be presented in the form of either a liquid formulation or a tablet formulation.

For example, when the above-mentioned peroxide component is the one capable of showing a pH of at least 9.0 when dissolved in water, e.g. when the peroxide component is sodium peroxide ($Na_2O_2$) or sodium hydrogen peroxide ($NaHO_2$), it may be presented in the form of a tablet containing such a component, as one type of the formulations for the cleaning agent of the present invention. In such a case, the user is capable of preparing a cleaning solution for contact lenses simply by dissolving such a tablet in a prescribed amount of water at the time of use.

Further, in a case where it is presented in the form of a liquid formulation, it may be provided as a liquid formulation containing peroxide ions and/or hydrogen peroxide ions and having the pH adjusted to a level of at least 9, so that the liquid formulation can be used as it is without any further adjustment. Otherwise, it may be formulated in a two part liquid formulation. In such a case, the user prepares a cleaning solution for contact lenses by diluting a stock solution containing the peroxide component with a controlling solution containing a pH controlling component at the time of use. It is advantageous to adopt such a two part liquid formulation in that the pH of the stock solution containing the peroxide component can be set at a level within a range where such a peroxide component can be stored under a most stable condition, whereby the cleaning ability can be maintained in good condition, and the pH value of the cleaning solution for contact lenses to be prepared can be made to a desired value of at least 9 by adjusting the amount of the controlling solution to be added.

To the cleaning agent of the present invention, various additives, such as a pH controlling agent and an osmotic pressure controlling agent, may of course be added, as the case requires. Such a pH-controlling agent may be the one which is useful also as the above-mentioned pH controlling component. As its specific examples, sodium hydroxide, dilute hydrochloric acid, a carbonate buffer, a phosphate buffer or a borate buffer may be mentioned. As the osmotic pressure controlling agent, an inorganic salt such as NaCl may be employed. Further, a chelating agent such as EDTA may also be incorporated. Further, a nonionic or anionic surfactant may of course be incorporated.

Now, the present invention will be described in further detail with reference to some Examples of the present invention. However, it should be understood that the present invention is by no means restricted by such specific Examples.

The recovered turbid soft contact lenses used in the following Examples were obtained by selecting and recovering those having protein soil deposited to form turbidity, among commercially available soft contract lenses composed mainly of HEMA and having a water content of 38%. Likewise, the recovered turbid oxygen permeable hard contact lenses were obtained by selecting and recovering those having protein soil deposited to form turbidity among commercially available oxygen permeable hard contact lenses. Accordingly, the periods of usage of such lenses are unknown.

When immersed in the cleaning solution of the present invention, soft contact lenses may undergo swelling or contraction to some extent. However, they can be restored to the original shapes by leaving them in a physiological saline solution, and no adverse effects to the physical properties of the lenses will remain.

With respect to the protein removal rates in the following Examples and Comparative Examples, the removal rates of protein soil were calculated from the area of the peak of the amide II band of the protein appearing around $1,550\ cm^{-1}$ in the FT-IR spectrum, in accordance with the following formula:

$$\text{Removal rate (\%)} = \frac{A-B}{A} \times 100$$

A: Area of the peak prior to the protein removal treatment
B: Area of the peak after the protein removal treatment

EXAMPLE 1

0.01 g of sodium peroxide was put into a 20 ml beaker, and cold water was added thereto to dissolve sodium peroxide and to bring the total amount to 10 ml. Then, the pH was adjusted to 12 with dilute hydrochloric acid to obtain a desired cleaning solution.

1 ml of the cleaning solution thus obtained, was put into a case for boiling a soft contact lens, and a recovered turbid soft contact lens was immersed therein and boiled for 20 minutes on hot water bath. Then, the lens was taken out and cleaned with a commercially available cleaning agent for contact lenses by means of a silicone puff. As a result of this cleaning treatment (once), 45% of the turbid protein soil was removed. Further, a similar treatment was repeated, and the removal rate became 95% after the second treatment to obtain a transparent cleaned lens.

EXAMPLE 2

0.01 g of sodium peroxide was put into a beaker in the same manner as in Example 1, and cold water was added to bring the total amount to 10 ml. Then, the pH was adjusted to 10 with dilute hydrochloric acid to obtain a cleaning solution.

Using this cleaning solution, the cleaning treatment was conducted in the same manner as in Example 1. By the first treatment, 22% of the turbid protein soil was removed. Further, a similar treatment was repeated, and the removal rate became 43% after the second treatment, 70% after the third treatment and 95% after the fourth treatment, to obtain a transparent cleaned lens.

EXAMPLE 3

0.01 g of sodium peroxide was put into a 200 ml beaker, and cold water was added to dissolve sodium peroxide and to bring the total amount to 100 ml. The pH of the solution thus obtained was measured and found to be pH11.4.

Using such a solution as the cleaning solution, the cleaning treatment was conducted in the same manner as in Example 1. By the first treatment, 23% of the turbid protein soil was removed. Further, a similar treatment was repeated, and the removal rate became 50% after the second treatment, 75% after the third treatment and 98% after the fourth treatment, to obtain a transparent cleaned lens.

EXAMPLE 4

A commercially available 31% hydrogen peroxide aqueous solution was diluted to obtain 10 ml of a 0.1% hydrogen peroxide aqueous solution. This solution was adjusted to pH12 with a 3N sodium hydroxide aqueous solution to obtain a desired cleaning solution.

Using this cleaning solution, the cleaning treatment was conducted in the same manner as in Example 1. By the first treatment, 74% of the turbid protein soil was removed to obtain a transparent cleaned lens.

EXAMPLE 5

A commercially available 31% hydrogen peroxide aqueous solution was diluted with a phosphate buffer solution to obtain an aqueous hydrogen peroxide solution having a concentration of 3.0% and pH3.0. Then, to 1 ml of this solution, 5 ml of a solution containing 0.148% of sodium carbonate, 0.155% of sodium hydrogen carbonate and 0.85% of sodium chloride, was added to obtain a hydrogen peroxide cleaning solution having a concentration of 0.5% and having pH9.5 and an osmotic pressure of 290 mmol/kg.

Using the cleaning solution thus obtained, the cleaning treatment was conducted in the same manner as in Example 1. By the first treatment, 91% of the turbid protein soil was removed to obtain a transparent cleaned lens.

EXAMPLE 6

To the hydrogen peroxide cleaning solution prepared in Example 5, 0.007% of a polypropylene oxide-polyethylene oxide block copolymer (polyethylene oxide content: 40% by weight, average molecular weight: 3,330) as the nonionic surfactant and 0.014% of a polypropylene oxide-polyethylene oxide block copolymer (polyethylene oxide content: 80% by weight, average molecular weight: 10,000), were added and incorporated.

Using the solution thus obtained, the cleaning treatment was conducted in the same manner as in Example 1. By the first treatment, 95% of the turbid protein soil was removed to obtain a transparent cleaned lens.

EXAMPLE 7

To the hydrogen peroxide cleaning solution prepared in Example 5, 0.01% of sodium dodecyl sulfate was added and incorporated.

Then, using this solution, the cleaning treatment was conducted in the same manner as in Example 1. By the first treatment, 92% of the turbid protein soil was removed to obtain a transparent cleaned lens.

EXAMPLE 8

To the hydrogen peroxide cleaning solution prepared in Example 5, 0.01% of cetyltrimethyl ammonium chloride was incorporated, and the cleaning treatment was conducted in the same manner as in Example 1. By the first treatment, 90% of the turbid protein soil was removed to obtain a transparent cleaned lens.

EXAMPLE 9

To the hydrogen peroxide cleaning solution prepared in Example 5, 0.05% of trisodium ethylene diamine tetraacetate was incorporated, and the pH was adjusted to 9.5. Then, the cleaning treatment was conducted in the same manner as in Example 1. By the first treatment, 98% of the turbid protein soil was removed to obtain a transparent cleaned lens.

EXAMPLE 10

To the hydrogen peroxide cleaning solution prepared in Example 5, 0.004% of a polypropylene oxide-polyethylene oxide block copolymer (polyethylene oxide content: 40% by weight, average molecular weight: 3,330) as the nonionic surfactant and 0.008% of a polypropylene oxide-polyethylene oxide block copolymer (polyethylene oxide content: 80% by weight, average molecular weight: 10,000), were added and incorporated.

Then, 1 ml of the solution thus obtained, was put in a case for boiling a contact lens, and a recovered turbid soft contact lens was immersed therein and heated on a hot water bath of 50°C for 5 minutes. Then, the lens was taken out and cleaned with a commercially available cleaning agent for soft contact lenses by means of a silicone puff. By the first treatment, 26% of the turbid protein soil was removed. Further, a similar treatment was repeated, and the removal rate became 55% after the second treatment and 85% after the third treatment, to obtain a transparent cleaned lens.

EXAMPLES 11 TO 13

The pH of the hydrogen peroxide cleaning solution prepared in Example 5 was adjusted with a 0.5N hydrochloric acid aqueous solution or a 0.5N sodium hydroxide aqueous solution to obtain cleaning solutions having pH9, pH10 and pHll, respectively.

Then, using the respective cleaning solutions, the cleaning treatment was conducted in the same manner as in Example 1. By the first treatment 94% of the protein soil was removed with the cleaning solution of pH9, and the removal rate was 82% with the cleaning solution of pH10 and 91% with the cleaning solution of pH11.

COMPARATIVE EXAMPLE 1

The hydrogen peroxide cleaning solution prepared in Example 5 was adjusted to pH7 with a 0.5N hydrochloric acid aqueous solution. By using the adjusted solution, the cleaning treatment was conducted in the same manner as in Example 1. By the first treatment, 19% of the turbid protein soil was removed. Further, a similar treatment was repeated, and the removal rate was 31% after the second treatment.

COMPARATIVE EXAMPLE 2

The hydrogen peroxide cleaning solution prepared in Example 5 was adjusted to pH3 with a 0.5N hydrochloric acid aqueous solution. Using this adjusted solution, the cleaning treatment was conducted in the same manner as in Example 1, whereby the protein soil was not removed at all, and a similar treatment was repeated three times, but the removal rate was unchanged at 0%.

COMPARATIVE EXAMPLE 3

Using a commercially available enzyme-containing cleaning agent ("Hydrocare F" tradename, manufactured by Allergan, INC.), a recovered turbid soft contact lens was immersed at room temperature for 6 hours, whereby the removal rate of the protein soil was 36%.

REFERENCE EXAMPLE

0.5 g of potassium carbonate was dissolved in 10 ml of a commercially available 3% hydrogen peroxide disinfectant ("Consept 1", tradename, manufactured by Barnes Hind, INC.) to obtain a desired cleaning solution. The pH of the solution thereby obtained was about 10.5. To the solution thus obtained, a recovered turbid soft contact lens was immersed and left to stand at room temperature for 20 minutes. Then, the lens was taken out and rinsed with a physiological saline solution while gently rubbing it.

COMPARATIVE EXAMPLE 4

A commercially available enzyme-containing cleaning agent ("Ultrazyme", tradename, manufactured by Allergan, INC.) was dissolved in 10 ml of the commercially available 3% hydrogen peroxide disinfectant as used in the Reference Example. Using this cleaning solution, a recovered turbid soft contact lens was subjected to the same cleaning treatment as in the Reference Example, whereby the protein soil was not removed at all.

As is evident from the foregoing results, when a contact lens is cleaned by the method of the present invention, the protein soil can be effectively removed in a very short period of time, and even the protein soil penetrated into the interior of the soft contact lens can be effectively decomposed and removed. Yet, there will be no discoloration problem with respect to colored contact lenses or soft contact lenses with markings. Therefore, the present invention is applicable advantageously also to such lenses.

**Claims**

1. A method for removing soil deposited on a contact lens, which comprises immersing the contact lens having soil such as protein soil deposited thereon, in an aqueous solution maintained at a temperature of at least 40°C containing peroxide ions and/or hydrogen peroxide ions at a concentration of from 0.00001 to 20.0% and having the pH adjusted to a level of at least 9.

**Patentansprüche**

1. Ein Verfahren zum Entfernen von Schmutz von Kontaktlinsen, welches das Eintauchen einer mit einer darauf abgeschiedenen Verschmutzung wie Protein verunreinigten Kontaktlinse in eine bei einer Temperatur von mindestens 40°C gehaltene, Peroxidionen und/oder Wasserstoffperoxidionen in einer Konzentration von 0.00001 bis 20.0 % enthaltende und auf einen pH von mindestens 9 eingestellte wäßrige Lösung umfaßt.

**Revendications**

1. Procédé pour enlever des souillures qui se sont déposées sur une lentille de contact, qui comprend l'immersion de la lentille de contact présentant des souillures telles que des dépôts protéiniques, dans une solution aqueuse maintenue à une température d'au moins 40°C contenant des ions peroxyde et/ou des ions peroxyde d'hydrogène, à une concentration de 0,00001 à 20,0 % et dont le pH est ajusté à un niveau d'au moins 9.